(19) Europäisches Patentamt / European Patent Office / Office européen des brevets

(11) **EP 2 200 701 B1**

(12) **EUROPEAN PATENT SPECIFICATION**


(45) Date of publication and mention of the grant of the patent:
**02.03.2011 Bulletin 2011/09**

(21) Application number: **08783474.3**

(22) Date of filing: **05.09.2008**

(51) Int Cl.:
***A61Q 13/00*** (2006.01) ***A61L 9/015*** (2006.01)
***C07C 43/13*** (2006.01) ***C07C 43/178*** (2006.01)
***C07C 59/13*** (2006.01) ***C07C 59/62*** (2006.01)
***C07C 69/675*** (2006.01) ***C07C 69/68*** (2006.01)

(86) International application number:
**PCT/CH2008/000374**

(87) International publication number:
**WO 2009/030059 (12.03.2009 Gazette 2009/11)**


(54) **DIMETHYLCYCLOHEXYL DERIVATIVES AS MALODOR NEUTRALIZERS**

DIMETHYLCYCLOHEXYL-DERIVATE ALS MITTEL ZUR NEUTRALISATION UNANGENEHMER GERÜCHE

DERIVES DE DIMETHYLCYCLOHEXYLE UTILISES COMME AGENTS NEUTRALISANT LES MAUVAISES ODEURS


(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.09.2007 US 970676 P**

(43) Date of publication of application:
**30.06.2010 Bulletin 2010/26**



(73) Proprietor: **Givaudan SA**
**1214 Vernier (CH)**

(72) Inventors:
- **FLACHSMANN, Felix**
**8600 Duebendorf (CH)**
- **GAUTSCHI, Markus**
**8117 Fällanden (CH)**
- **SGARAMELLA, Richard, P.**
**Hoboken, New Jersey 07030 (US)**
- **MCGEE, Thomas**
**Chittering, W.A. 6084 (AU)**

(74) Representative: **Sievert, Claudia**
**Givaudan Schweiz AG**
**Global Patents**
**Überlandstrasse 138**
**CH-8600 Dübendorf (CH)**



(56) References cited:
**EP-A- 1 167 507** **EP-A- 1 262 474**
**EP-A- 1 398 366** **EP-A- 1 459 735**
**WO-A-00/14051** **WO-A-2004/050602**
**FR-A- 2 008 167** **GB-A- 1 159 188**
**US-A- 4 187 251** **US-A- 4 622 221**
**US-A- 5 166 412**



Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).


EP 2 200 701 B1

## Description

The present invention refers to malodor neutralizing compounds and to compositions containing them.

Malodors are offensive odors which are ubiquitous. They have either personal or environmental origin. For example, sweat, urine and feces malodors are personal in origin, whereas kitchen and cooking malodors or the malodor of cold tobacco smoke are of environmental origin. While personal malodors are easily deposited on fabric, hair, and skin, environmental malodors also have a propensity to deposit on these substrates. The term "malodor" is used to refer to smells or odors generally regarded as undesirable or unpleasant in nature.

Amines, thiols, sulfides, short chain aliphatic and olefinic acids, e.g. fatty acids, are typical of the chemicals found in and contributing to sweat, household, and environmental malodors. These types of malodors typically include indole, skatole, and methanethiol found in toilet and animal odours; piperidine and morpholine found in urine; pyridine and triethyl amine found in kitchen and garbage odors; and short chain fatty acids, such as 3-methyl-3-hydroxyhexanoic acid, 3-methylhexanoic acid or 3-methyl-2-hexenoic acid, found in axilla malodors. Compounds which have been found in the axilla are described for example by Xiao-Nong Zeng et al., Journal of Chemical Ecology, Vol. 17, No. 7, 1991 page 1469 -1492.

The most commonly employed route to reducing malodors is the use of aroma chemicals possessing a pleasant strong odor to mask them. This approach is somewhat limited as it is often found that the composition over time loses its malodor control effectiveness. In addition, aroma chemicals are not very effective against malodors associated with nitrogen and/or sulfur compounds. These malodors are particularly difficult to mask as they have a very low perception threshold. Another problem with conventional aromatic chemicals in the form of liquids or solids is that such compounds tend to evaporate over an extended period of time, which may result in the return of the malodor.

US 4,187,251, US 4,622,221 and EP 1 167 507 disclose compounds for counteracting malodors.

Other approaches to reducing malodors are the elimination by absorption of the malodor by a porous or cage-like structure often in combination with aroma chemicals, and avoidance of the formation of malodors by such routes as antimicrobials and enzyme inhibitors. While these approaches have yielded improvements in malodor control, there still remains a need for further compounds which are even more efficient against malodors.

Surprisingly, the inventors now found a new class of compounds that are capable of neutralizing malodors, in particular they are very efficient against nitrogen- and sulfur-based malodors.

Accordingly, the present invention refers in a first aspect to the use as malodor neutralizer of a compound of formula (I)

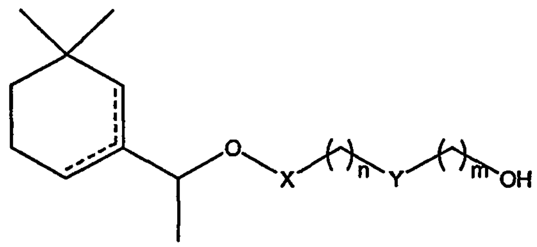

**(I)**

wherein the dotted line is no bond or the dotted line together with the carbon-carbon bond represents a double bond;
X and Y represent independently carbonyl, $-CH_2-$, -CHMe-, or $-C(Me)_2-$; and
n and m are independently 0 or 1, e.g. n = m = 0;
with the proviso that if X is carbonyl either or both of n or m is 0.

Further examples are those compounds of formula (I) wherein the dotted line is no bond, and n = m = 0.

Further examples are those compounds of formula (I) wherein n = m = 0, X is carbonyl and Y is $-CH_2-$, or -CHMe-.

Further examples are those compounds of formula (I) wherein n = m = 0, Y is carbonyl and X is $-CH_2-$, or -CHMe-.

Particularly preferred are compounds of formula (I) for the use as a malodor neutralizer selected from the group consisting of
2-(1-(3,3-dimethylcyclohexyl)ethoxy)-2-methylpropan-1-ol,
1-(3,3-dimethylcyclohexyl)ethyl 2-hydroxypropanoate*,
2-(1-(3,3-dimethylcyclohexyl)ethoxy)acetic acid,
2-(1-(3,3-dimethylcyclohexyl)ethoxy)ethanol*
3-(1-(3,3-dimethylcyclohexyl)ethoxy)-3-methylbutan-1-ol,
3-(1-(3,3-dimethylcyclohexyl)ethoxy)-3-methylbutanoic acid,
2-(1-(3,3-dimethylcyclohexyl)ethoxy)propanoic acid,
2-(1-(3,3-dimethylcyclohex-1-enyl)ethoxy)acetic acid,

2-(1-(5,5-dimethylcyclohex-1-enyl)ethoxy)acetic acid,
2-(1-(3,3-dimethylcyclohex-1-enyl)ethoxy)propanoic acid,
2-(1-(5,5-dimethylcyclohex-1-enyl)ethoxy)propanoic acid,
1-(3,3-dimethylcyclohexyl)ethyl 2-hydroxyacetate,
1-(3,3-dimethylcyclohex1-enyl)ethyl 2-hydroxyacetate,
1-(5,5-dimethylcyclohex-1-enyl)ethyl 2-hydroxyacetate,
1-(3,3-dimethylcyclohex1-enyl)ethyl 2-hydroxypropanoate,
1-(5,5-dimethylcyclohex1-enyl)ethyl 2-hydroxypropanoate,
2-(1-(3,3-dimethylcyclohex1-enyl)ethoxy)ethanol,
2-(1-(5,5-dimethylcyclohex1-enyl)ethoxy)ethanol,
2-(1-(3,3-dimethylcyclohexyl)ethoxy)propan-1-ol,
2-(1-(3,3-dimethylcyclohex1-enyl)ethoxy)propan-1-ol,
2-(1-(5,5-dimethylcyclohex-1-enyl)ethoxy)propan-1-ol,
1-(1-(3,3-dimethylcyclohexyl)ethoxy)propan-2-ol
1-(1-(3,3-dimethylcyclohex-1-enyl)ethoxy)propan-2-ol
1-(1-(5,5-dimethylcyclohex-1-enyl)ethoxy)propan-2-ol
3-(1-(3,3-dimethylcyclohexyl)ethoxy)butan-2-ol,
3-(1-(3,3-dimethylcyclohex-1-enyl)ethoxy)butan-2-ol, and
3-(1-(5,5-dimethylcyclohex-1-enyl)ethoxy)butan-2-ol, and mixtures thereof.

Even more preferred are those compounds of formula (I) indicated by * in the list above which possess little odor or neutral odors so that they may also be used in consumer products that contain fragrance without distorting the fragrance.

The term "malodor neutralizer" as used herein means the interaction of a compound of formula (I) that can interact with malodor compounds, such that the effect on the human sense of smell is the alleviation of the offensiveness of the malodor. However, it is not intended that this term be limited to any particular mechanism by which such a result may be obtained.

The compounds of formula (I) may be added to a wide variety of consumer products, such as household products, personal care products and cosmetics, both fragranced and perfume-free.

Household products which may comprise a compound as defined hereinabove include air fresheners in the form of aerosols, sprays, liquids, solids, gels and powders, which may be used e.g. in cars, apartments, food containers, as garbage pail deodorants and refrigerator fresheners, or in large-scale closed air systems such as auditoriums, subways and transport systems; cleansers such as disinfectants, hard surface cleaners, toilet bowl cleaners; effluent control products such as sewage treatment, garbage disposal treatments; softeners, fabric refreshers and scourers.

Personal care products and cosmetics, which may comprise a compound as defined hereinabove include oral products, such as toothpaste, mouthwashes, breath-freshening strips and candies; bathroom accessories such as paper towels, bathroom tissues; personal hygiene products such as sanitary napkins, panty liners, incontinence pads, disposable wash clothes, disposable diapers, and diaper pail deodorants; antiperspirant and underarm deodorants, general body deodorants in the form of powders, aerosols, liquids or solid, or hair care products such as hair sprays, conditioners, rinses, hair colors and dyes, permanent waves, depilatories, hair straighteners, hair groom applications such as pomades, creams, lotions, etc., medicated hair care products or shampoos, or foot care products such as foot powders, liquids, or colognes, after shaves and body lotions, or soaps and synthetic detergents such as bars, liquids, foams, or powders.

The compounds of the present invention may also be used in products for odor control in agriculture such as pig and hen house effluents, and pet care products such as domestic animal litter. The compounds may also be used in products such as paint and gasoline.

Typically the consumer product comprises from about 0.001% to about 90% by weight, e.g. about 0.01, 0.1, 1, 10 or 20% by weight, of at least one compound of formula (I) as hereinabove defined, based on the end-product. The effective amount depends on the type of product into which the compound of formula (I) or a mixture thereof is admixed. For example, if used in an air freshener consisting of essentially 100% volatile components, the product may comprise about 10% by weight of a compound of formula (I), or a mixture thereof, based on the end-product. If used in detergent products the end-product may comprise about 0.05% by weight, or if used in fabric refresher products, i.e. products that restore freshness to fabrics and clothing, the product may comprise about 0.1 to 0.5% by weight of a compound of formula (I) or a mixture thereof.

Accordingly, the present invention refers in a further aspect to a consumer product comprising an effective malodor neutralizing amount of a compound of formula (I), or a mixture thereof, as hereinabove described.

In a further aspect the present invention refers to a method of enhancing the malodor neutralizing properties of consumer products, comprising the step of admixing to a consumer product, e.g. personal care product, household product or cosmetic product, a compound of formula (I) as defined hereinabove, or a mixture thereof.

The compounds of formula (I) when used in fragranced consumer products are preferably combined with

fragrances which are known to be effective as deodorant, such as aldehyde C10, heliotropin (3,4-methylenedioxybenzaldehyde), benzyl cinnamate, tolyl aldehyde, cinnamic aldehyde, alpha-amyl-cinnamic aldehyde, alpha-hexyl-cinnamic aldehyde, vanillin, benzaldehyde, cuminic aldehyde (4-isopropylbenzaldehyde), heliotropin, tolyl aldehyde, anisyl acetate, benzyl salicylate, dihydro eugenol, geraniol, methyl eugenol, para cresyl methyl ether, styrallyl acetate, anisic aldehyde, o-allyl-vanillin, ethyl-aubepin (4-ethoxybenzaldehyde), ethyl-vanillin, carvacrol, 5-methyl-2-(2-methylpropyl)-1,3-dioxane, methyl 1,4 dimethylcyclohexyl-carboxylate, iso-Eugenol, Elintaal (acetaldehyde ethyl linalyl acetal), Dispirone (7,9-dimethylspiro[5,5]undecanone-3), 4-tert-amyl cyclohexane, p-t-butyl-alpha-methyl hydrocinnamic aldehyde, 2-n-heptyl cyclopentanone, alpha cetone (3-methyl-4-(2,6,6-trimethylcyclohex-2-enyl)but-3-en-2-one), beta-methyl naphthyl ketone, cyclohexadecanolide, ethylene brassylate, cyclopentadecanolide, cyclopentadecanone, nonanediol-1,3-diacetate, nonanolide-1,4 (5-pentyldihydrofuran-2(3H)-one), iso-nonyl acetate, nopol acetate (2-(6,6-dimethylbicyclo [3.1.1]hept-2-en-2-yl)ethyl acetate), Dimyrcetol (mixture of 2,6-dimethyloct-7-enol-2 & formate), phenylethyl alcohol, tetrahydro muguol (mixture of 3,7-dimethyloctanol-3 & 2,6-dimethyloctanol-2), 4-tert-butylcyclohexyl acetate, iso-amyl salicylate, clove leaf oil, benzoin siam resinoids, bergamot oil, geranium oil, patchouli oil, petitgrain oil and thyme oil, or mixtures thereof.

However the compounds of formula (I) may also be composed with any other fragrance raw material selected from the extensive range of natural and synthetic molecules, such as extracts, essential oils, absolutes, resinoids, resins, concretes etc., but also synthetic materials such as hydrocarbons, alcohols, aldehydes, ketones, ethers, acids, esters, acetals, ketals, nitriles, etc., including saturated and unsaturated compounds, aliphatic, carboxcyclic, and heterocyclic compounds. These compounds are known to the person skilled in the art and are described e.g. in Arctander, Perfume and Flavor Chemicals (Montclair, NJ., 1969), in S. Arctander, Perfume and Flavor Materials of Natural Origin (Elizabeth, N.J., 1960) and in "Flavor and Fragrance Materials-1991", Allured Publishing Co. Wheaton, III. USA.

The compounds of formula (I) may also be used in combination with other malodor counteractants such as other malodor neutralizers, malodor absorbers, and/or preventive ingredients.

By "preventative ingredient" is meant an antimicrobial or enzyme inhibitor that prevents the malodor from forming. Non-limiting examples of antimicrobial agents that may be used in conjunction with the compounds of formula (I) are bacteriostatic quaternary ammonium compounds, such as 2-amino-2-methyl-1-propanol, cetyl-trimethylammonium bromide, cetyl pyridinium chloride, 2, 4, 4'-trichloro-2'-hydroxydiphenylether, N-(4-chlorophenyl)-N'-(3,4-dichlorophenyl)urea, silver halides, octoxyglycerin and various zinc salts such as zinc citrate, zinc oxide, zinc riconoleate, zinc pyrethiones, and octopirox; parabens, such as methyl paraben, propyl paraben, butyl paraben, ethyl paraben, isopropyl paraben, isobutyl paraben, benzyl paraben, and their salts; alcohols, such as benzyl alcohol, phenyl ethyl alcohol; boric acid; phenolic compounds, such as phenol, 2-methyl phenol, 4-ethyl phenol; essential oils such as rosemary, thyme, lavender, eugenol, geranium, tea tree, clove, lemon grass, peppermint, or their active components such as anethole, thymol, eucalyptol, farnesol, menthol, limonene, methyl salicylate, salicylic acid, terpineol, nerolidol, geraniol, and mixtures thereof. Non-limiting examples of enzyme inhibitors are glutaraldehyde, p-hydroxybenzaldehyde, phthalic dicarboxyaldehyde, and benzaldehyde; salts containing ions selected from the group consisting of silver, zinc, and copper; complexes containing ions selected from the group consisting of silver, zinc, and copper; organic bromo-nitro compounds, such as 2-bromo-2-nitro-1,3 propanedial; phosphoamide compounds, such as phenyl phosphorodiamidate; and quinones, diethyl- and dimethyl-cyclohex-2-en-1-one, citronellol, 2-methyl-3-(4-(1-methylethyl)phenyl)propanal, (2-(methyloxy)-4-propyl-1-benzenol), diphenylmethane, tetrahydrolinalol, 4-(4-methyl-3-pentenyl)cyclohex-3-ene-1-carbaldehyde, 3-(4-methyl-3-pentenyl)cyclohex-3-ene-1 carbaldehyde, 3-(1,3-benzodioxol-5-yl)-2-methylpropanal, alpha-ionone, beta-ionone, tricyclo[5.2.1.0,2,6]dec-4-en-8-yl ethanoate, 4-(4-hydroxy-4-methylpentyl)cyclohex-3-enecarbaldehyde, 3-(4-hydroxy-4-methylpentyl)-cyclohex-3-enecarbaldehyde, methyl iso-eugenol, 2-(1,1-dimethylethyl)cyclohexyl ethanoate, 4-(1,1-dimethylethyl)cyclohexyl ethanoate, and 4-methyl-2-(2-methylprop-1-enyl)tetrahydropyran.

Malodor neutralizers which may be combined with the compound of formula (I) include cycloalkyl tertiary alcohols such as cyclohexyl methanol, 1-cyclohexyl-1-ethanol, 1-cyclohexyl propanol, esters of alpha beta unsaturated monocarboxylic acids such as tiglic acid geranyl ester, senecioic acid geranyl ester, methyl crotonate, and octyl crotonate, and mixtures thereof, fumaric acid esters such as dialkylene esters of fumaric acid, such as digeranyl fumarate, dihexyl fumarate, dibenzyl fumarate, and mixtures thereof, derivatives of acetic and propionic acids such as 2-hydroxyethyl phenoxyacetate, 2-hydroxyethyl p-tert-butylphenoxyacetate, 2'-hydroxyethyl 3-phenoxypropionate, and 4-cyclohexyl-4-methyl-2-pentanone, and mixtures thereof.

By "malodor absorber" is meant an odor-absorbing material that can eliminate a broad spectrum of odoriferous molecules by physical adsorption or entrapment. Examples include activated charcoal, zeolites, ground corn cob and cyclodextrins.

Solvents are also frequently used with fragrances and may be selected from many classes of low-odor volatile compounds that are known in the art, for example, ethers, such as dimethyl ether, diethyl ether, and mixtures thereof; straight or branched chain alcohols and diols, such as methanol, ethanol, propanol, isopropanol, n-butyl alcohol, t-butyl alcohol, benzyl alcohol, butoxypropanol, butylene glycol, and isopentyldiol (3-methylbutane-1,2-diol), and mixtures thereof; volatile silicones, such as cyclomethicones for example octamethyl cyclo tetrasiloxane and decamethyl cyclopentane

siloxane, and mixtures thereof; dipropylene glycol, triethyl citrate, ethanol, isopropanol, diethyleneglycol monoethyl ether, diethyl phthalate, triethyl citrate, isopropyl myristate, hydrocarbon solvents, such as Isopar® available from ExxonMobile Chemicals, other known solvents that have previously been used to dispense volatile actives from substrates.

Whereas compounds such as 2-(1-(3,3-dimethylcyclohexyl)ethoxy)-2-methylpropan-1-ol, i.e. a compound of formula (I) wherein X is $-C(Me)_2-$, Y is $-CH_2-$, and n = m = 0, and 1-(l-(3,3-dimethylcyclohexyl)ethoxy)-2-methylpropan-2-ol, i.e. a compound of formula (I) wherein n = m 0, X is $-CH_2-$, and Y is $-C(Me)_2-$, are disclosed in US 5,166,412 as an intermediate for the production of fragrance compounds, others have never been described in the literature.

Accordingly, the present invention refers in a further aspect to compounds of formula (I)

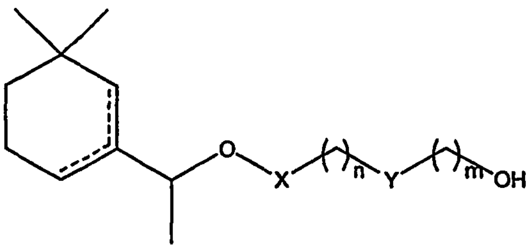

(I)

wherein the dotted line is no bond or the dotted line together with the carbon-carbon bond represents a double bond; X is carbonyl, or $-C(Me)_2-$; and Y is carbonyl, $-CH_2-$, -CHMe-, or$-C(Me)_2-$; or X and Y is $-CH_2-$; n and m are independently 0 or 1, e.g. n = m = 0; with the proviso that

- if n and m is 0 and X is $-C(Me)_2-$ then Y is not $-CH_2-$;
- if X is carbonyl then either or both of n or m is 0; and
- the coumpound of formula (I) is not (1S,1 'R)-1-(3',3'-dimethyl-1'-cyclohexyl)ethyl hydroxyacetate.

The compounds of formula (I) comprise at least one chiral centre and as such they may exist as a mixture of enantiomers and diastereomers, or they may be resolved as enantiomerically and diastereomerically pure forms. However, resolving steroisomers adds to the complexity of manufacture and purification of these compounds and so it is preferred to use a compound of formula (I) as a mixture of its stereoisomers simply for economic reasons. However, if it is desired to prepare pure stereoisomers, this may be achieved according to methodology known in the art. Accordingly the present invention refers in a further aspect to a compound of formula (I) in the form of anyone of its isomers or a mixture thereof.

The compounds of the present invention may be prepared by reaction of cyclademol (1-(3,3-dimethylcyclohexy) ethanol) and its unsaturated analogs respectively with $A-X-(CH_2)_n-Y-(CH_2)_m-OH$ (A being a leaving group, such as a hydroxyl, chloride, bromide or fluoride, an alkanoyloxy group or an arylsulfonyloxy group) by a carbon-oxygen bond forming reaction, such as the Williamson ether synthesis or an esterification. Thereby, the hydroxyl group of $A-X-(CH_2)_n-Y-(CH_2)_m-OH$ highlighted in bold may need to be protected by a protective group (PG), for example a silyl ether, a benzyl ether, or a tetrahydropyranyl ether. The procedures for introduction and removal of such protective groups are known to the person skilled in the art of organic synthesis. They are described for example in T. W. Greene, P. G. M. Wuts, Protective Groups in Organic Synthesis, John Wiley & Sons, New York 1999. Preparation modes for various groups of compounds of the present invention are described more in detail in the following.

The compounds of formula (I) wherein X is carbonyl may be formed via an esterification reaction with a carboxylic acid derivative III as depicted in Scheme 1. For compounds of formula III wherein A = -OH, the reaction is carried out in the presence of a catalyst such as p-toluene sulfonic acid or sulfuric acid under removal of water, typically in a Dean-Stark apparatus. For compounds of formula III wherein A is chloride, bromide or fluoride, the reaction is carried out in the presence of a suitable base, typically a trialkylamine or pyridine. Suitable solvents, temperatures, times and workup conditions are known to the person skilled in the art of organic synthesis.

Scheme 1:

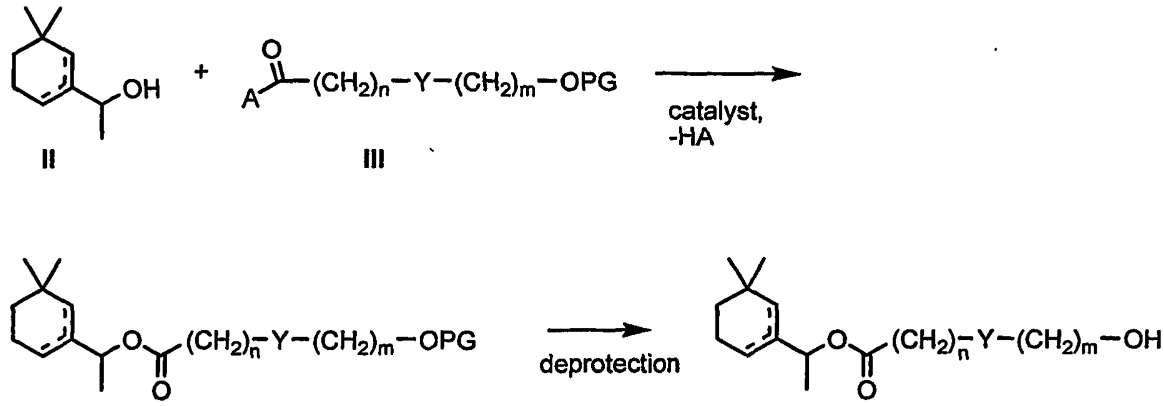


For compounds of formula (I) wherein X is a $-CH_2-$ or a -CHMe- group, the compounds of the present invention may be formed via an etherification reaction (Williamson synthesis) with a reagent IV as depicted in Scheme 2. This reaction is typically performed with a halide as leaving group, i.e. a reagent IV wherein A is selected from chloride, bromide and iodide, in a solvent such as tetrahydrofurane, dimethyl formamide or dimethyl sulfoxide and in the presence of a suitable base, such as sodium hydride, potassium hydroxide or potassium carbonate.

Scheme 2:

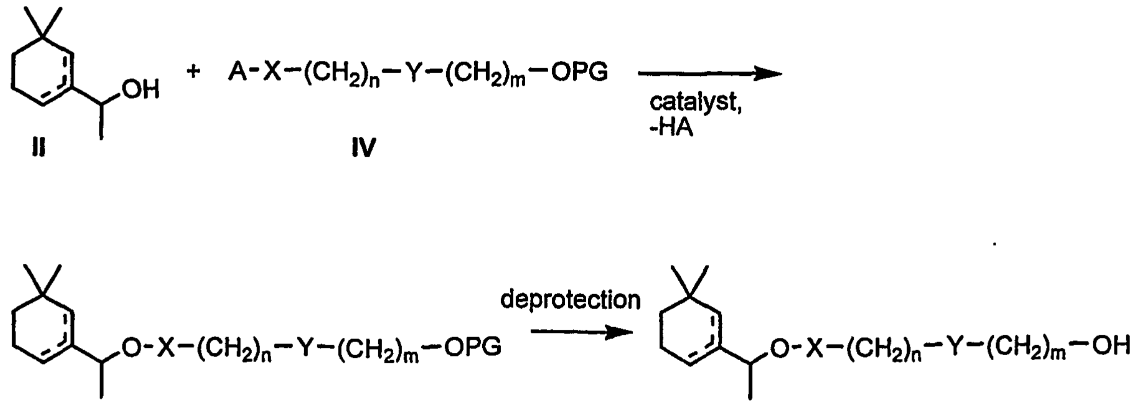


Compounds of formula (I) wherein X is a $-C(Me)_2-$ group, the above ether synthesis as depicted in Scheme 2 might be inefficient and such products may be synthesized by catalytic opening of a corresponding oxacycle, such as a substituted oxirane or a oxacyclobutane.

The invention is now further described with reference to the following examples.

Example 1: 1-(3,3-dimethylcyclohexyl)ethyl 2-hydroxypropanoate

1-(3,3-dimethylcyclohexyl)ethanol (50.0 g, 0.32 mol), lactic acid (37.3 g, .352 mol, 1.1 equiv.) and *p*-toluenesulfonic acid (3.04 g, 16 mmol, 5 mol%) are dissolved in toluene (320 ml) and the solution is refluxed in a flask equipped with a Dean-Stark apparatus for 1.5 h. During this period, 11 ml of $H_2O$ are collected. After cooling to room temperature, the solution is washed with sat. aq. $NaHCO_3$-solution, then dried over $MgSO_4$. The crude pale yellow liquid obtained after removal of the solvent is purified by distillation (91°C, 0.07 mbar) to yield 40.8 g (56%) of a colourless oil as a mixture of 4 diastereomers.

$^{13}C$-NMR (100 MHz, $CDCl_3$): 175.5 (s), 175.4 (s), 76.6 (d), 76.5 (d), 66.9 (d), 66.6 (d), 41.2 (t), 39.0 (t), 39.0 (t), 38.4 (d), 38.3 (d), 33.5 (q), 33.5 (q), 30.5 (s), 30.5 (s), 28.4 (t), 28.3 (t), 24.6 (q), 21.9 (t), 21.9 (t), 20.6 (q), 20.5 (q), 17.1 (q), 17.0 (q).

Example 2: 2-(1-(3,3-dimethylcyclohexyl)ethoxy)acetic acid

1-(3,3-dimethylcyclohexyl)ethanol (20.0 g, 0.128 mol) are added dropwise to the stirred suspension of NaH (6.8 g of a 55% suspension in mineral oil, 0.153 mol) in toluene (50 ml) over 10 min at room temperature. After heating to reflux for 20 h, the suspension is cooled to room temperature and a solution of chloroacetic acid (6.0 g, 0.064 mol, 0.5 equiv.) in toluene (40 ml) is added dropwise over 20 min. The resulting mixture is heated again to reflux for 48 h then cooled to room temperature and hydrolyzed with 2 N aq. HCl solution. The aqueous layer is extracted with MTBE and the combined organic layers are washed with $H_2O$ and dried over $MgSO_4$. The crude pale yellow liquid obtained after removal of the solvent is purified by distillation over a Vigreux column (110°C, 0.09 mbar) to yield 10.6 g (77 %) of a colourless, viscous oil, consisting of 2 diastereomers in a 5:2 ratio.

$^{13}$C-NMR (100 MHz, $CDCl_3$, main isomer): 175.4 (s), 81.6 (d), 65.5 (t), 41.8 (t), 39.2 (t), 38.6 (d), 33.6 (q), 30.6 (s), 28.0 (t), 24.6 (q), 22.0 (t), 16.0 (q).

Example 3: 2-(1-(3,3-dimethylcyclohexyl)ethoxy)ethanol

The suspension of $NaBH_4$ (6.10 g, 162.2 mmol, 3.3 equiv in dimethoxyethane (100 ml) is cooled to 0°C and treated dropwise with titanium tetrachloride (5.9 ml, 54.1 mmol, 1.1 equiv.). After 10 min stirring, the solution of 2-(1-(3,3-dimethylcyclohexyl)ethoxy)acetic acid (10.53 g, 49.1 mmol, 1 equiv.) in dimethoxyethane (50 ml) is added dropwise during 20 min, and the resulting mixture is stirred at room temperature for 20 h. After hydrolysis with water/ice and extraction with MTBE, the organic layers are combined and washed with $H_2O$, then dried over $MgSO_4$. The crude oil obtained after removal of the solvent is purified by FC on $SiO_2$ and the purified product is bulb-to-bulb distilled (88°C, / 0.08 mbar) to yield 7.0 g (71%) of product as a colourless liquid, which consists to 84% of a mixture of two diastereomers in a 3:1 ratio, besides 16% of an inseparable constitutional isomer.

$^{13}$C-NMR (100 MHz, $CDCl_3$): 80.5 (d), 80.4 (d), 69.8 (t), 69.7 (t), 62.1 (t), 41.9 (t), 41.5 (t), 39.3 (t), 39.3 (t), 39.0 (t), 38.9 (d), 33.6 (q), 31.7 (q), 30.6 (q), 29.7 (t), 28.8 (t), 28.2 (t), 24.7 (d), 24.6 (t), 22.1 (t), 22.1 (q), 16.5 (q), 16.4 (q).

Example 4: Malodor reduction efficiency against hexyl amine and allyl disulphide mercaptan.

Amines are key contributors to many household malodors. Sulfides are present in odors associated with bad breath. In order to assess the malodor reduction efficacy of the compounds of the present invention, they were evaluated for their ability to reduce hexyl amine malodor and allyl disulphide by the following method.

A one liter glass headspace collection jar had placed inside it a 25 ml stoppered glass container which contained 0.5 g of a test compound. 10 ul of hexyl amine or allyl disulphide mercaptan as a representative malodor was injected into the headspace jar. This was left for 15 minutes at 25°C to equilibrate. One ml/minute of the headspace was drawn for one minute through a Tenax™ headspace trap. The Tenax™ trap was removed and thermally desorbed into an Agilent 6890 GC/MS to analyze the quantity of hexyl amine and allyl disulphide mercaptan respectively in the headspace to determine the initial concentration of the hexyl amine malodor/ally disulphide mercaptan. The glass stopper was removed from the container and the test component and the malodor were left in contact for 60 minutes at 25°C. One ml/minute of the headspace was drawn for one minute through a Tenax™ trap and the amount of malodor remaining was determined as above. The results are shown in Table 1 below:

Table 1. Malodor Reduction

| Compound | % Reduction of hexyl amine | % Reduction of allyl disulphide |
|---|---|---|
| A | 80.3 | 81.5 |
| B | 91.4 | 74.6 |
| C | 71.2 | 70.2 |

where

Compound A = 1-(3,3-dimethylcyclohexyl)ethyl 2-hydroxypropanoate
Compound B = 2-(1-(3,3-dimethylcyclohexyl)ethoxy)acetic acid
Compound C = 2-(1-(3,3-dimethylcyclohexyl)ethoxy)ethanol

where

$$\% \text{ Reduction} = \frac{100 \text{ (headspace of malodor (ng/l) - headspace of mixture (ng/l))}}{\text{headspace of malodor (ng/l)}}$$

As can be seen from the results in Table 1, the compounds of formula (I) showed good reduction of the malodor of both, hexyl amine and allyl disulphide.

Example 5: Evaluation of the malodor reduction efficiency of kitchen malodors

Evaluation of the malodor reduction potential of the compounds of the present invention on malodors of natural garlic oil and onion oil using the methodology described in Example 4 is shown in Table 2. As standard for determining the reduction of onion oil odor the amount of diallyl sulfide and diallyl disulfide were measured, and propyl methyl disulfide and dipropyl disulfide for determining the reduction of garlic oil odor.

Table 2: Malodor reduction efficiency against garlic oil and onion oil

| Compound | % Reduction of onion oil odor | % Reduction of garlic oil odor |
|---|---|---|
| A | 87.2 | 87.7 |
| B | 66.3 | 73.2 |
| C | 78.4 | 67.4 |

The compounds of the present invention display satisfactory malodor reduction properties and are suitable for consumer products that are used to mitigate kitchen odors.

Example 6: Comparison against a commercially available malodor neutralizer

A 0.1% ethanol solution of synthetic axilla malodor was prepared and a 100ul aliquot was placed onto cotton pads. 1 % solutions of test compounds were prepared and 100ul dosed on to the axilla treated pads. One pad was left untreated as the control. Each pad was allowed to dry for 15 minutes. The test cotton pads were randomized, and a panel of 20 was used. Each panelist was asked to check a box that represented the strength of the axilla malodor using a Labeled Magnitude Scale (LMS) (Barry G. Green, Pamela Dalton, Beverly Cowart, Greg Shaffer, Krystyna Rankin and Jennifer Higgins. Evaluating the Labeled Magnitude Scale for Measuring Sensations of Taste and Smell. Chemical Senses. Vol. 21, pp 323-334, 1996).

The % reduction of malodor was calculated by:

$$\% \text{ Reduction} = \frac{100 \text{ (Mean LMS (Control) - Mean LMS (test solution))}}{\text{Mean LMS (Control)}}$$

The results are shown in Table 3.

Table 3: Percent reduction of axilla malodor:

| Compound | % Reduction of Axilla |
|---|---|
| citronellyl methylcrotonate (comparison) | 53.1 |
| A | 77.2 |
| B | 67.6 |
| C | 61.4 |

The compounds of the present invention are significantly better at reducing axilla odor than the commercial malodor neutralizer Sinodor (citronellyl methylcrotonate). They are thus suitable for products that help reduce body odors on the skin or articles of clothing.

Example 7: Fragrance composition

| Ingredient | % (w/w) |
|---|---|
| Benzyl Alcohol | 5.00 |
| Cinnamyl Alcohol | 5.00 |
| Dihydromyrcenol | 6.00 |
| Diphenyl Oxide | 2.50 |
| Heliotropin | 1.00 |
| Citronellyl Acetate | 5.00 |
| Indole | 0.10 |
| Hedione | 10.00 |
| Phenyl Ethyl Alcohol | 5.75 |
| Myrascone | 2.50 |
| Linalyl Butyrate | 5.00 |
| Mayol | 10.50 |
| Terpineol | 16.60 |
| Vanillin | 0.05 |

Composition 1: fragrance composition according to the formula above plus 25% (w/w) of diethyl phthalate, a low odor solvent.

Composition 2: fragrance composition according to the formula above plus 10% (w/w) of diethyl phthalate and 15% (w/w) 1-(3,3-dimethylcyclohexyl)ethyl 2-hydroxypropanoate (compound A).

Composition 1 was compared with composition 2 for malodor reduction efficiency using the method described in Example 6. The results are shown below.

| | % reduction of axilla malodor |
|---|---|
| Composition 1 | 70.0 |
| Composition 2 | 81.1 |

As can be seen from the figures above, the malodor reduction of Composition 2 is significant superior to the malodor reduction effect of Composition 1, i.e. a fragrance composition comprising pleasant odorants only.

Example 8: Air freshener products for reduction of malodor in the air

A) Plug-in Liquid Electrical Air Freshener

Composition 1 and Composition 2 (of Example 7), respectively, were diluted with solvent 3-methoxy-3-methyl-1-butanol in a 75:25 ratio composition: solvent. They were placed in commercial glass refill bottles into which wicks were inserted through a vented collar. The refills were assembled in an appropriate electrical air freshener heater unit. The units were left for 24 hours to equilibrate. Into two 183 cubic foot (5.18 $m^3$) stainless steel smelling booths Petri-dishes containing a 2x2 inch (5x5cm) cotton pad dosed with 0.2g of a 10% standard GSA Bathroom malodor solution (from General Services Administration Federal Supply Service) were placed on a table on the back wall. These were left for 45 minutes. The units were plugged into the back wall and left for 45 minutes. Each unit had a 3 digit randomly generated code. A panel of 10 trained assessors was used. The panelists were asked to compare the booths and select the one with the lowest level of malodor. The order of presentation was randomized. Nine out of 10 panelists selected the booth in which Composition 2 was placed.

B) Gel-Based Air Fresheners.

Two air freshener gels were prepared with one with Composition 1 (see Example 7) and one with Composition 2 (see Example 7) as follows:

| Ingredients | Suppliers | % by Weight |
|---|---|---|
| Lithene N4-9000-MA10 | Synthomer | 16.75 |
| Composition 1 or 2 | Givaudan SA | 75.15 |
| LX-10096 Pylakrome Red Dye | Pylam Co. | 0.10 |
| Steol CS-460 | Stepan | 5.00 |
| Jeffamine D-400 | Hunstman | 3.00 |

The gels were placed in Petri dishes and left to equilibrate for 24 hours. Into two 183 cubic feet (5.18 $m^3$) stainless steel smelling booth a lit cigarette was placed in an ashtray and allowed to bum for 5 minutes. After 5 minutes, the cigarette was extinguished and removed.

Immediately after the cigarette and ashtray were removed, the Petri dish containing the respective gel was placed on a table set against the back wall of the booth. After 45 minutes a panel of 10 trained assessors was asked to compare each booth for malodor intensity. The panelists were asked to select the booth with the lowest intensity of malodor. The gels were evaluated in random order. Eight out of 10 selected the booth in which the air freshener gel containing Composition 2 was placed.

## Claims

1. Use as malodor neutralizer of a compound of formula (I)

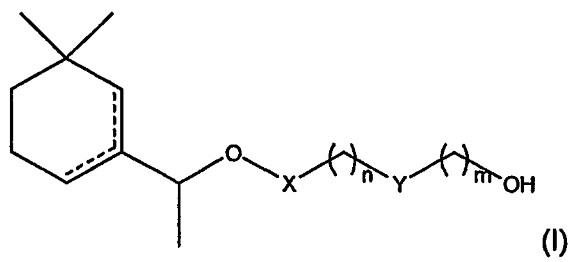

(I)

wherein
the dotted line is no bond or the dotted line together with the carbon-carbon bond represents a double bond;
X and Y represent independently carbonyl, $-CH_2-$ -CHMe-, or $-C(Me)_2-$; and n and m are independently 0 or 1;
with the proviso that if X is carbonyl either or both of n or m is 0.

2. Use according to claim 1 wherein the compound of formula (I) is selected from the group consisting of 2-(1-(3,3-dimethylcyclohexyl)ethoxy)-2-methylpropan-1-ol, 1-(3,3-dimethylcyclohexyl)ethyl 2-hydroxypropanoate, 2-(1-(3,3-dimethylcyclohexyl)ethoxy)-acetic acid, 2-(1-(3,3-dimethylcyclohexyl)ethoxy)ethanol, 3-(1-(3,3-dimethylcyclohexyl)ethoxy)-3-methylbutan-1-ol, 3-(1-(3,3-dimethylcyclohexyl)ethoxy)-3-methylbutanoic acid, 2-(1-(3,3-dimethylcyclohexyl)ethoxy)propanoic acid, 2-(1-(3,3-dimethylcyclohex-1-enyl)ethoxy)acetic acid, 2-(1-(5,5-dimethylcyclohex-1-enyl)ethoxy)acetic acid, 2-(1-(3,3-dimethylcyclohex-1-enyl)ethoxy)propanoic acid, 2-(1-(5,5-dimethylcyclohex-1-enyl)ethoxy)propanoic acid, 1-(3,3-dimethylcyclohexyl)-ethyl 2-hydroxyacetate, 1-(3,3-dimethylcyclohex1-enyl)ethyl 2-hydroxyacetate, 1-(5,5-dimethylcyclohex-1-enyl)ethyl 2-hydroxyacetate, 1-(3,3-dimethylcyclohex1-enyl)ethyl 2-hydroxypropanoate, 1-(5,5-dimethylcyclohexl-enyl)ethyl 2-hydroxypropanoate, 2-(1-(3,3-dimethylcyclohex1-enyl)ethoxy)ethanol, 2-(1-(5,5-dimethylcyclohex1-enyl)ethoxy)ethanol, 2-(1-(3,3-dimethylcyclohexyl)ethoxy)propan-1-ol, 2-(1-(3,3-dimethylcyclohexl-enyl)ethoxy)propan-1-ol, 2-(1-(5,5-dimethylcyclohex-1-enyl)ethoxy)propan-1-ol, 1-(1-(3,3-dimethylcyclohexyl)ethoxy)propan-2-ol, 1-(1-(3,3-dimethylcyclohex-1-enyl)ethoxy)propan-2-ol, 1-(1-(5,5-dimethylcyclohex-1-enyl)ethoxy)propan-2-ol, 3-(1-(3,3-dimethylcyclohexyl)ethoxy)butan-2-ol, 3-(1-(3,3-dimethylcyclohex-1-enyl)ethoxy)butan-2-ol, 3-(1-(5,5-dimethylcyclohex-1-enyl)ethoxy)butan-2-ol, and mixtures thereof.

3. A compound of formula (I)

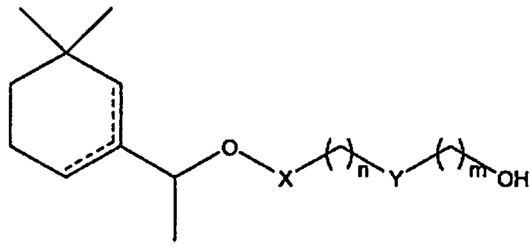

(I)

wherein the dotted line is no bond or the dotted line together with the carbon-carbon bond represents a double bond;
X is carbonyl, or $-C(Me)_2-$; and
Y is carbonyl, $-CH_2-$, -CHMe-, or $-C(Me)_2-$; or
X and Y is $-CH_2-$; and
n and m are independently 0 or 1;
with the proviso that

- if n and m is 0 and X is $-C(Me)_2-$ then Y is not $-CH_2-$;
- if X is carbonyl then either or both of n or m is 0;
- the compound of formula (I) is not (1S. 1'R)-1-(3',3'-dimethyl-1'-cyclohexyl)ethyl hydroxyacetate.

**4.** A method of enhancing the malodor neutralizing properties of consumer products, comprising admixing to a consumer product a compound of formula (I) as defined in claim 1, or a mixture thereof.

**5.** A method of neutralizing malodor from the air or from surfaces, comprising applying thereto an effective amount of a compound of formula (I) as defined in claim 1, or a mixture thereof.

**6.** A fragrance composition comprising at least one compound of formula (I) as defined in claim 1 and at least one fragrance raw material.

**7.** A consumer product comprising a compound of formula (I) as defined in claim 1, or a mixture thereof.

**8.** A product according to claim 7 wherein the consumer product is selected from the group of household products, personal care products and cosmetics.

**Patentansprüche**

**1.** Verwendung einer Verbindung der Formel (I)

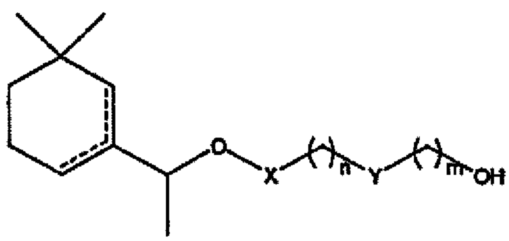

(I)

worin
die gestrichelte Linie für keine Bindung steht oder die gestrichelte Linie zusammen mit der Kohlenstoff-Kohlenstoff-Bindung für eine Doppelbindung steht;
X und Y unabhängig voneinander für Carbonyl, $-CH_2-$, -CHMe- oder $-C(Me)_2-$ stehen und
n und m unabhängig voneinander für 0 oder 1 stehen;
mit der Maßgabe, daß dann, wenn X für Carbonyl steht, n und/oder m für 0 stehen;
als Mittel zur Neutralisation unangenehmer Gerüche.

2. Verwendung nach Anspruch 1, wobei die Verbindung der Formel (I) aus der Gruppe bestehend aus 2-(1-(3,3-Dimethylcyclohexyl)ethoxy)-2-methyl-propan-1-ol, 2-Hydroxypropansäure-(1-(3,3-dimethylcyclohexyl)-ethyl)ester, 2-(1-(3,3-Dimethylcyclohexyl)ethoxy)essigsäure, 2-(1-(3,3-Dimethylcyclohexyl)ethoxy)ethanol, 3-(1-(3,3-Dimethylcyclohexyl)ethoxy)-3-methylbutan-1-ol, 3-(1-(3,3-Dimethylcyclohexyl)ethoxy)-3-methyl-butansäure, 2-(1-(3,3-Dimethylcyclohexyl)ethoxy)propansäure, 2-(1-(3,3-Dimethylcyclohex-1-enyl)ethoxy)essigsäure, 2-(1-(5,5-Dimethylcyclohex-1-enyl)ethoxy)essigsäure, 2-(1-(3,3-Dimethylcyclohex-1-enyl)ethoxy)propansäure, 2-(1-(5,5-Dimethylcyclohex-1-enyl)ethoxy)propansäure, 2-Hydroxyessigsäure-1-(3,3-dimethylcyclohexyl)-ethylester, 2-Hydroxyessigsäure-1-(3,3-dimethylcyclohex-1-enyl)ethylester, 2-Hydroxyessigsäure-1-(5,5-dimethylcyclohex-1-enyl)ethylester, 2-Hydroxypropansäure-1-(3,3-dimethylcyclohex-1-enyl)ethylester, 2-Hydroxypropansäure-1-(5,5-dimethylcyclohex-1-enyl)ethylester, 2-(1-(3,3-Dimethylcyclohex-1-enyl)ethoxy)ethanol, 2-(1-(5,5-Dimethylcyclohex-1-enyl) ethoxy)ethanol, 2-(1-(3,3-Dimethylcyclohexyl)ethoxy)propan-1-ol, 2-(1-(3,3-Dimethylcyclohex-1-enyl)ethoxy)propan-1-ol, 2-(1-(5,5-Dimethylcyclohex-1-enyl)ethoxy)propan-1-ol, 1-(1-(3,3-Dimethylcyclohexyl)ethoxy)propan-2-ol, 1-(1-(3,3-Dimethylcyclohex-1-enyl)ethoxy)propan-2-ol, 1-(1-(5,5-Dimethylcyclohex-1-enyl)ethoxy)propan-2-ol, 3-(1-(3,3-Dimethylcyclohexyl)ethoxy)butan-2-ol, 3-(1-(3,3-Dimethylcyclohex-1-enyl)ethoxy)butan-2-ol, 3-(1-(5,5-Dimethylcyclohex-1-enyl)ethoxy)butan-2-ol
und Mischungen davon
ausgewählt ist.

3. Verbindung der Formel (I)

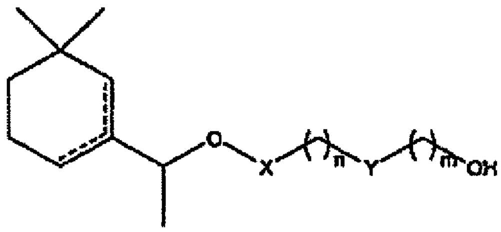

(I)

worin
die gestrichelte Linie für keine Bindung steht oder die gestrichelte Linie zusammen mit der Kohlenstoff-Kohlenstoff-Bindung für eine Doppelbindung steht;
X für Carbonyl oder $-C(Me)_2-$ steht und
Y für Carbonyl, $-CH_2-$, -CHMe- oder $-C(Me)_2-$ steht oder
X und Y für $-CH_2-$ stehen; und
n und m unabhängig voneinander für 0 oder 1 stehen;
mit der Maßgabe, daß

- dann, wenn n und m für 0 stehen und X für $-C(Me)_2-$ steht, Y nicht für $-CH_2-$ steht;
- dann, wenn X für Carbonyl steht, n und/oder m für 0 stehen;
- es sich bei der Verbindung der Formel (I) nicht um Hydroxyessigsäure-(1S,1'R)-1-(3',3'-dimethyl-1'-cyclohexyl) ethylester handelt.

4. Verfahren zur Verbesserung der Eigenschaften zur Neutralisation unangenehmer Gerüche von Verbraucherprodukten, bei dem man einem Verbraucherprodukt eine Verbindung der Formel (I) gemäß Anspruch 1 oder eine Mischung davon beimischt.

5. Verfahren zur Neutralisation unangenehmer Gerüche aus der Luft oder von Oberflächen, bei dem man darauf eine wirksame Menge einer Verbindung der Formel (I) gemäß Anspruch 1 oder einer Mischung davon aufbringt.

6. Duftstoffzusammensetzung, umfassend mindestens eine Verbindung der Formel (I) gemäß Anspruch 1 und mindestens einen Duftstoff-Rohstoff.

7. Verbraucherprodukt, umfassend eine Verbindung der Formel (I) gemäß Anspruch 1 oder eine Mischung davon.

8. Produkt nach Anspruch 7, wobei das Verbraucherprodukt aus der Gruppe Haushaltsprodukte, Körperpflegeprodukte und Kosmetika ausgewählt ist.

## Revendications

1. Utilisation en tant que neutralisant de mauvaises odeurs d'un composé de formule (I)

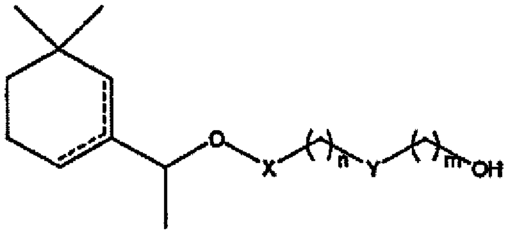

(I)

dans laquelle
la ligne en pointillés n'est pas une liaison ou la ligne en pointillés conjointement avec la liaison carbone-carbone représente une double liaison ;
X et Y représentent indépendamment carbonyle, $-CH_2-$, -CHMe-, ou $-C(Me)_2-$ ; et
n et m sont indépendamment 0 ou 1 ;
à condition que si X est carbonyle, l'un et/ou l'autre de n et m est O.

2. Utilisation selon la revendication 1 **caractérisée en ce que** le composé de formule (I) est choisi dans le groupe constitué de 2-(1-(3,3-diméthylcyclohexyl)éthoxy)-2-méthylpropan-1-ol, 2-hydroxypropanoate de 1-(3,3-diméthylcyclohexyl)éthyle, acide 2-(1-(3,3-diméthylcyclohexyl)éthoxy)-acétique, 2-(1-(3,3-diméthylcyclohexyl)éthoxy)éthanol, 3-(1-(3,3-diméthylcyclohexyl)éthoxy)-3-méthylbutan-1-ol,
acide 3-(1-(3,3-diméthylcyclohexyl)éthoxy)-3-méthylbutanoïque,
acide 2-(1-(3,3-diméthylcyclohexyl)éthoxy)propanoïque,
acide 2-(1-(3,3-diméthylcyclohex-1-ényl)éthoxy)acétique,
acide 2-(1-(5,5-diméthylcyclohex-1-ényl)éthoxy)acétique,
acide 2-(1-(3,3-diméthylcyclohex-1-ényl)éthoxy)propanoïque,
acide 2-(1-(5,5-diméthylcyclohex-1-ényl)éthoxy)propanoïque,
2-hydroxyacétate de 1-(3,3-diméthylcyclohexyl)-éthyle,
2-hydroxyacétate de 1-(3,3-diméthylcyclohex1-ényl)éthyle,
2-hydroxyacétate de 1-(5,5-diméthylcyclohex-1-ényl)éthyle,
2-hydroxypropanoate de 1-(3,3-diméthylcyclohexl-ényl)éthyle,
2-hydroxypropanoate de 1-(5,5-diméthylcyclohexl-ényl)éthyle, 2-(1-(3,3-diméthylcyclohexl-ényl)éthoxy)éthanol, 2-(1-(5,5-diméthylcyclohexl-ényl)éthoxy)éthanol, 2-(1-(3,3-diméthylcyclohexyl)éthoxy)propan-1-ol, 2-(1-(3,3-diméthylcyclohexl-ényl)éthoxy)propan-1-ol, 2-(1-(5,5-diméthylcyclohex-1-ényl)éthoxy)propan-1-ol, 1-(1-(3,3-diméthylcyclohexyl)éthoxy)propan-2-ol, 1-(1-(3,3-diméthylcyclohex-1-ényl)éthoxy)propan-2-ol, 1-(1-(5,5-diméthylcyclohex-1-ényl)éthoxy)propan-2-ol, 3-(1-(3,3-diméthylcyclohexyl)éthoxy)butan-2-ol, 3-(1-(3,3-diméthylcyclohex-1-ényl)éthoxy)butan-2-ol,
3-(1-(5,5-diméthylcyclohex-1-ényl)éthoxy)butan-2-ol, et des mélanges de ceux-ci.

3. Composé de formule (I)

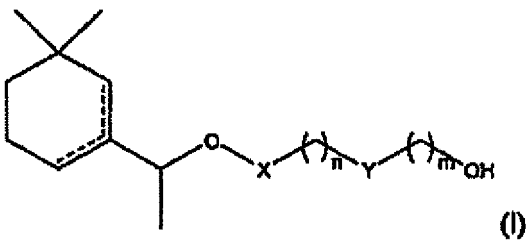

(I)

dans laquelle la ligne en pointillés n'est pas une liaison ou la ligne en pointillés conjointement avec la liaison carbone-carbone représente une double liaison ;
X est carbonyle, ou $-C(Me)_2-$ ; et
Y est carbonyle, $-CH_2-$, -CHMe-, ou $-C(Me)_2-$ ; ou

X et Y sont $-CH_2-$ ; et
n et m sont indépendamment 0 ou 1 ;
à condition que

- si n et m sont 0 et X est $-C(Me)_2-$, alors Y n'est pas $-CH_2-$ ;
- si X est carbonyle, alors l'un et/ou l'autre de n et m est 0 ;
- le composé de formule (I) n'est pas l'hydroxyacétate de (15,1'R)-1-(3',3'-diméthyl-1'-cyclohexyl)éthyle.

**4.** Méthode d'amélioration des propriétés neutralisantes des mauvaises odeurs de produits de grande consommation, comprenant le mélange d'un produit de grande consommation avec un composé de formule (I), tel que défini dans la revendication 1, ou un mélange de celui-ci.

**5.** Méthode de neutralisation des mauvaises odeurs de l'air ou des surfaces, comprenant l'application à ceux-ci d'une quantité efficace d'un composé de formule (I) tel que défini dans la revendication 1, ou d'un mélange de celui-ci.

**6.** Composition de parfum comprenant au moins un composé de formule (I) tel que défini dans la revendication 1 et au moins une matière première de parfum.

**7.** Produit de grande consommation comprenant un composé de formule (I) tel que défini dans la revendication 1 ou un mélange de celui-ci.

**8.** Produit selon la revendication 7, **caractérisé en ce que** le produit de grande consommation est choisi dans le groupe comprenant les produits ménagers, les produits d'hygiène personnelle et les produits cosmétiques.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4187251 A
- US 4622221 A
- EP 1167507 A
- US 5166412 A


### Non-patent literature cited in the description


- **Xiao-Nong Zeng et al.** *Journal of Chemical Ecology,* 1991, vol. 17 (7), 1469-1492
- **Arctander.** *Perfume and Flavor Chemicals,* 1969
- **S. Arctander.** *Perfume and Flavor Materials of Natural Origin,* 1960
- **T. W. Greene ; P. G. M. Wuts.** Protective Groups in Organic Synthesis. John Wiley & Sons, 1999
- **Barry G. Green ; Pamela Dalton ; Beverly Cowart ; Greg Shaffer ; Krystyna Rankin ; Jennifer Higgins.** Evaluating the Labeled Magnitude Scale for Measuring Sensations of Taste and Smell. *Chemical Senses,* 1996, vol. 21, 323-334